(19) Europäisches Patentamt
European Patent Office
Office européen des brevets

(11) Veröffentlichungsnummer: **0 614 970 A2**

# (12) EUROPÄISCHE PATENTANMELDUNG

(21) Anmeldenummer: **94102631.2**

(22) Anmeldetag: **22.02.94**

(51) Int. Cl.[5]: **C12N 9/02**, C07K 3/02, C07K 3/12, C12Q 1/26, C07C 49/76, C07D 239/24, C07D 207/00, A01N 35/04, A01N 35/06, A01N 37/10, A01N 37/18, A01N 37/34, A01N 41/00, A01N 43/36, A01N 43/48, A01N 47/06

(30) Priorität: **25.02.93 DE 4305696**

(43) Veröffentlichungstag der Anmeldung:
**14.09.94 Patentblatt 94/37**

(84) Benannte Vertragsstaaten:
**CH DE FR GB IT LI**

(71) Anmelder: **Hoechst Schering AgrEvo GmbH**
**Gerichtstrasse 27**
**D-13342 Berlin (DE)**

(72) Erfinder: **Schulz, Arno, Dr.**
**In den Amtmannswiesen 5**
**D-65817 Eppstein (DE)**

## (54) Inhibitoren der Hydroxyphenylpyruvat-Dioxygenase sowie Nachweisverfahren zur Identifizierung des Enzyms.

(57) Es wird ein Verfahren zur Anreicherung der anabolen p-Hydroxyphenylpyruvat-Dioxygenasen aus pflanzlichen Geweben beschrieben sowie ein Verfahren mit dem die enzymatische Aktivität des Enzyms einfach gemessen werden kann, ohne daß das Enzym völlig gereinigt werden muß.

Die Erfindung beschreibt außerdem ein Testsystem zur Identifizierung von Inhibitoren der p-Hydroxyphenylpyruvat-Dioxygenase aus Pflanzen, in dem eine angereicherte HPPD aus Pflanzen mit einem zu untersuchenden Testsubstrat inkubiert wird und die enzymatische Aktivität des Enzyms im Vergleich zur Aktivität des nicht gehemmten Enzyms ermittelt wird.

Die Erfindung beschreibt außerdem die nach dem erfindungsgemäßen Testverfahren gefundenen neuen herbiziden Wirkstoffe der allgemeinen Formel (I).

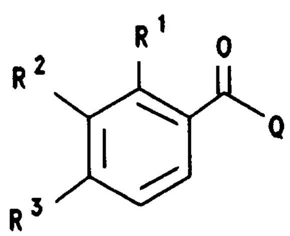

(I)

EP 0 614 970 A2

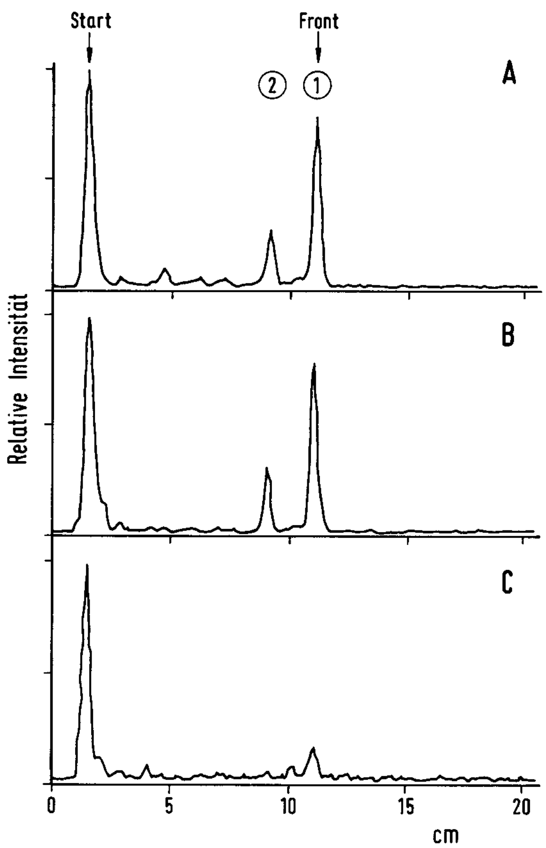


Fig. 1

p-Hydroxyphenylpyruvat-Dioxygenase (HPPD; p-hydroxyphenylpyruvate : oxygen oxidoreductase, EC 1.13.11.27) ist in pflanzlichen Geweben ein zentrales Enzym in der Biosynthese der von der Aminosäure Tyrosin abgeleiteten chinoiden Verbindungen wie Plastochinone oder Tocopherole. Im Verlauf der Chinonbiosynthese wird Tyrosin zunächst von einer Transaminase zu p-Hydroxyphenylpyruvat transaminiert, welches dann von dem Enzym HPPD in einer komplexen Reaktionsfolge decarboxyliert und hydroxyliert wird. Das Produkt dieser Reaktion ist Homogentisinsäure. Diese ersten beiden Schritte der Chinonbiosynthese sind in analoger Weise auch in tierischen Geweben und Mikroorganismen nachweisbar. Während in Pflanzen aber die gebildete Homogentisinsäure zu Plastochinonen und Tocopherolen umgesetzt werden kann, ist sie in Tieren und Mikroorganismen ein Intermediat im Katabolismus der Aminosäure Tyrosin. Plastochinone und Tocopherole sind für Pflanzen lebensnotwendige Bausteine. Inhibitoren der Biosynthese von Plastochinonen und Tocopherolen sollten daher potentielle Herbizide sein.

Katabole p-Hydroxyphenylpyruvat-Dioxygenasen wurden bisher aus tierischen Geweben (Lindstedt, S. und Odelhög, B. (1987) Meth. Enzymol. 142, 139 - 142) und Mikroorganismen (Lindstedt, S. und Odelhög, B. (1987) Meth. Enzymol. 142, 143-148) gereinigt und charakterisiert. Pflanzliche (anabole) p-Hydroxyphenylpyruvat-Dioxygenasen wurden dahingegen zwar in der Literatur beschrieben (Fiedler, E., Soll, J. und Schultz, G. (1982) Planta 155, 511 - 515), aber bisher wurde keine Methode zur Anreicherung des Enzyms beschrieben.

Speziell fehlen in der Literatur bisher Angaben über einfache Meßverfahren des pflanzlichen Enzyms, welche naturgemäß für eine Reinigung des Enzyms und für den Nachweis von Inhibitoren des Enzyms unerläßlich sind.

Das von Fiedler et al. beschriebene Verfahren ist relativ aufwendig und gestattet es nicht, die zur Identifizierung von potentiellen Herbiziden notwendigen quantitativen Tests durchführen zu können.

Es wird im folgenden ein Verfahren zur Anreicherung der anabolen p-Hydroxyphenylpyruvat-Dioxygenasen aus pflanzlichen Geweben beschrieben sowie ein Verfahren mit dem die enzymatische Aktivität des Enzyms einfach gemessen werden kann, ohne daß das Enzym völlig gereinigt werden muß.

Die Erfindung betrifft somit:

Ein Verfahren zur Anreicherung einer anabolen p-Hydroxyphenylpyruvat-Dioxygenase aus Pflanzenzellen, dadurch gekennzeichnet, daß man das Enzym direkt aus einem Puffer isoliert, mit dem die Zellen homogenisiert wurden.

Die Erfindung betrifft außerdem ein Testsystem zur Identifizierung von Inhibitoren der p-Hydroxyphenylpyruvat-Dioxygenase aus Pflanzen, dadurch gekennzeichnet, daß man eine angereicherte HPPD aus Pflanzen mit einem zu untersuchenden Testsubstrat inkubiert und die enzymatische Aktivität des Enzyms im Vergleich zur Aktivität des nicht gehemmten Enzyms ermittelt.

Die Erfindung betrifft insbesondere ein Verfahren, in dem pflanzliche Gewebe oder Zellen mit Extraktionspuffer homogenisiert werden, der Extrakt filtriert wird, der Überstand einer fraktionierten Ammoniumsulfatfällung unterzieht und das entstandene Präzipitat wieder löst.

Als Extraktionspuffer kommen die für Pflanzenzellen üblichen Extraktionspuffer in Frage, insbesondere der Extraktionspuffer mit der folgenden Zusammensetzung:

20 mM Phosphatpuffer pH 7,0;
0,14 M KCl;
0,1 mg/ml Glutathion und
1 % unlösliches Polyvinylpyrolidon.

Als Testsubstrat kommen alle Verbindungen in Frage, die die HPPD möglicherweise inhibieren. Diese potentiellen Hemmstoffe haben vorzugsweise eine strukturelle Ähnlichkeit zu dem natürlichen Substrat des HPPD. Es können aber auch andere Substanzen verwendet werden, die nicht im aktiven Zentrum des Enzyms binden, sondern das Enzym auf andere Art und Weise hemmen.

Die Ermittlung der enzymatischen Aktivität kann mit den in der Literatur bekannten Maßnahmen durchgeführt werden (siehe Bergmeyer, H.U., Methoden der enzymatischen Analyse, Band 1 und 2, Verlag Chemie, Weinheim, (1974) und Suelter, C.H., Experimentelle Enzymologie: Grundlagen für die Laborpraxis, Fischer Stuttgart (1990)).

Die Meßmethode ist in abgewandelter Form für katabole HPPD's aus der menschlichen Leber von Lindblad (Lindblad, B. (1971) Clin. Chim. Acta 34, 113 - 121) beschrieben worden. Dabei wird in Endpunkt-Messungen $^{14}C$-$CO_2$ bestimmt, das durch die enzymatische Aktivität der HPPD aus $^{14}C$-p-Hydroxyphenylpyruvat freigesetzt wird. Sind in dem Reaktionsansatz Inhibitoren der HPPD vorhanden, so unterbleibt die Enzymreaktion und somit die Freisetzung von $CO_2$. Somit können mit dieser Testmethode Inhibitoren des Enzyms gefunden werden.

Vorzugsweise wird der Test in der Art durchgeführt, daß die enzymatische Aktivität der HPPD nach einer Vorinkubation der angereicherten HPPD mit dem potentiellen Hemmstoff durch die Zugabe des

radioaktiv markiertem $^{14}C$-p-Hydroxyphenylpyruvat gestartet wird, die Reaktion nach einer geeigneten Inkubationszeit abgebrochen wird und die enzymatische Aktivität indirekt über die freigesetzte Radioaktivität gemessen wird.

Der erfindungsgemäße Test kann selbstverständlich auch mit dem gereinigten Enzym ausgeführt werden. Das Enzym kann in an sich bekannter Weise durch Chromatographie an Anionentauschern, wie z. B. Q-Sepharose der Firma Pharmacia und anschließende Gelpermeationschromatographie, wie z.B. Superdex 200 von Pharmacia weiter angereichert werden, dies ist jedoch für die Durchführung des Tests nicht notwendig.

Überraschenderweise wurde mit diesem Testsystem nun auch eine neue Klasse von Inhibitoren der HPPD identifiziert. Dies sind Herbizide aus der Gruppe der 2-Benzoyl-Cyclohexan-1,3-Dione. Dies ist überraschend, weil für diese Verbindungsklasse aufgrund ihrer herbiziden Symptomatik - sie führen zum Ausbleichen der Pflanzen - in der Literatur ein völlig anderer Wirkmechanismus diskutiert wird. Es wird vermutet, daß sie, analog zu anderen bleichenden Herbiziden, die Phytoendesaturase hemmen (Soeda, T. und Uchida, T. (1987) Pestic. Biochem.Physiol. 29, 35 - 42; Mayonada, D.J., Hatzios, K.K., Orcutt, D.M. and Wilson, H.P. (1989) Pestic. Biochem. Physiol. 35, 138 - 145). Es konnte gezeigt werden, daß diese Verbindungsklasse nicht die Phytoendesaturase, wohl aber die HPPD hemmt; unter den Testbedingungen wurde die anabole pflanzliche HPPD aus Mais von 4,5 x $10^{-8}$ M 2-(2-chloro-4-methansulfonylbenzoyl)-1-3-cyclohexandion (SC-0051, ein typischer Vertreter dieser Herbizidklasse) zu 50 % gehemmt (entspricht dem IC50-Wert). Bleichende Herbizide mit einer anderen Struktur hemmen die HPPD dagegen nicht, sondern inhibieren das Enzym Phytoendesaturase.

Mit dem erfindungsgemäßen Verfahren kann aber nicht nur die Wirkung bekannter Herbizide auf molekularer Ebene nachgewiesen werden, es können auch neue, bisher nicht bekannte herbizide Wirkstoffe identifiziert werden, die die HPPD inhibieren.

Die Erfindung betrifft daher auch die nach dem erfindungsgemäßen Testverfahren gefundenen neuen herbiziden Wirkstoffe.

Gegenstand der Erfindung sind daher auch Verbindungen der Formel (I)

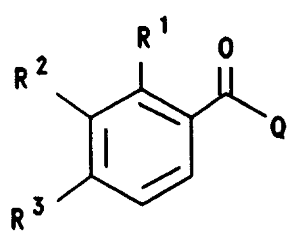

(I)

worin

- $R^1$ Wasserstoff, Halogen, OH, Alkoxy, CN, $NO_2$ oder Halogenalkyl,
- $R^2$ Wasserstoff, Halogen, OH, Alkyl, Alkoxy, Halogenalkyl, Halogenalkoxy oder (Alkoxy)-carbonyl,
- $R^3$ Wasserstoff, Halogen, OH, CN, $NO_2$, Halogenalkyl, Halogenalkoxy oder $R^4S(O)_m$-, wobei $R^4$ Alkyl und m null, eins oder zwei sind,
- Q einen Rest aus der Gruppe mit der Formel Q1 bis Q5,

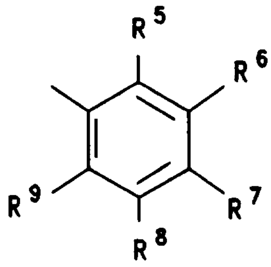 , 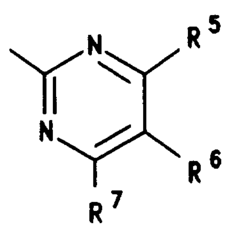 , 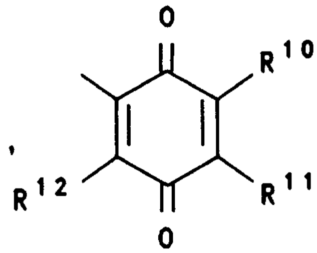

(Q1) (Q2) (Q3)

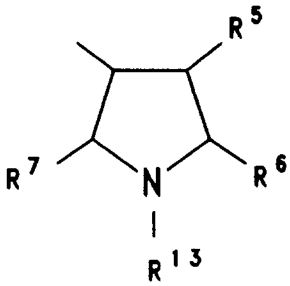 oder 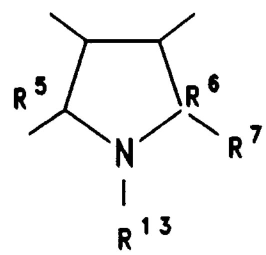

(Q4) (Q5)

wobei

- $R^5$, $R^6$, $R^7$, $R^8$ und $R^9$ unabhängig voneinander H, Halogen, OH, CN, $NO_2$, $SO_3H$, $SO_3R^4$, $SO_2NR^{14}R^{15}$, COOH, $COOR^4$, $CONR^{14}R^{15}$, O-$COOR^4$, O-$COR^4$, Alkyl, Alkoxy, Halogenalkyl oder Halogenalkoxy,
- $R^{10}$, $R^{11}$ und $R^{12}$ unabhängig voneinander Wasserstoff, Halogen, Alkyl, Alkoxy, Halogenalkyl oder Halogenalkoxy,
- $R^{13}$ Wasserstoff, Phenylsulfonyl, Alkylsulfonyl oder Alkylcarbonyl,
- $R^{14}$ und $R^{15}$ unabhängig voneinander Wasserstoff, Alkyl, Aryl oder Benzyl bedeuten.

In der Formel (I) können die Reste Alkyl, Alkoxy, Halogenalkyl und Halogenalkoxy jeweils geradkettig oder verzweigt sein. Alkylreste bedeuten beispielsweise Methyl, Ethyl, n- oder i-Propyl, n-, i-, t- oder 2-Butyl.

Aryl umfasst aromatische und heteroaromatische Reste wie beispielsweise Phenyl, 2- oder 3-Naphthyl, 2-, 3- oder 4-Pyridyl, Halogen bedeutet Fluor, Chlor, Brom oder Iod.

Von besonderem Interesse ist die erfindungsgemäße Verwendung von Verbindungen der Formel (I), worin

- $R^1$ Wasserstoff, Halogen, OH, $(C_1-C_3)$-Alkoxy, CN, $NO_2$ oder $(C_1-C_3)$-Halogenalkyl,
- $R^2$ Wasserstoff, Halogen, OH, $(C_1-C_3)$-Alkyl, $(C_1-C_3)$-Alkoxy, $(C_1-C_2)$-Halogenalkyl, $(C_1-C_2)$-Halogenalkoxy oder $(C_1-C_2$-Alkoxy)-carbonyl,
- $R^3$ Wasserstoff, Halogen, OH, CN, $NO_2$, $(C_1-C_3)$-Halogenalkyl, $(C_1-C_2)$-Halogenalkoxy oder $R^4S(O)_m$-, wobei $R^4$ $(C_1-C_2)$-Alkyl und m null, eins oder zwei sind,
- Q einen Rest aus der Gruppe mit der Formel

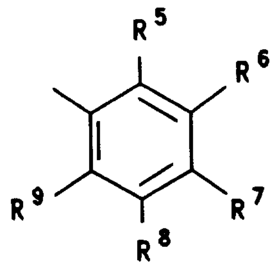
,
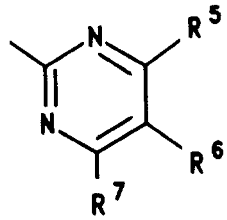
,
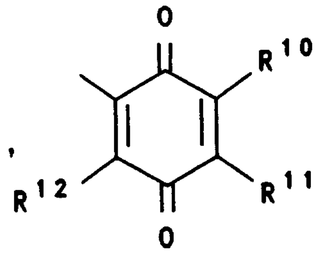

(Q1) (Q2) (Q3)

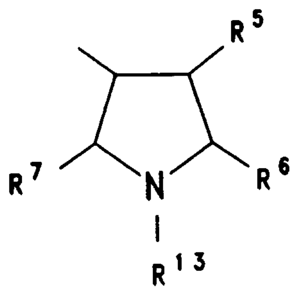

oder

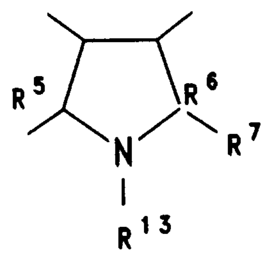

(Q4) (Q5)

wobei

$R^5$, $R^6$, $R^7$, $R^8$ und $R^9$ unabhängig voneinander H, Halogen, OH, CN, $NO_2$, $SO_3H$, $SO_3R^4$, $SO_2NR^{14}R^{15}$, COOH, $COOR^4$, $CONR^{14}R^{15}$, O-$COOR^4$, O-$COR^4$-, ($C_1$-$C_4$)-Alkyl, ($C_1$-$C_2$)-Alkoxy, ($C_1$-$C_3$)-Halogenalkyl oder ($C_1$-$C_2$)-Halogenalkoxy,

$R^{10}$, $R^{11}$ und $R^{12}$ unabhängig voneinander Wasserstoff, Halogen, ($C_1$-$C_3$)-Alkyl, ($C_1$-$C_2$)-Alkoxy, ($C_1$-$C_3$)-Halogenalkyl oder ($C_1$-$C_2$)-Halogenalkoxy,

$R^{13}$ Wasserstoff, Phenylsulfonyl, ($C_1$-$C_2$)-Alkylsulfonyl oder ($C_1$-$C_2$)-Alkylcarbonyl,

$R^{14}$ und $R^{15}$ unabhängig voneinander Wasserstoff, ($C_1$-$C_4$)-Alkyl, Phenyl oder Benzyl, bedeuten.

Bevorzugt ist die erfindungsgemäße Verwendung von Verbindungen der Formel (I), worin mindestens eines der folgenden Merkmale enthalten ist:

$R^1$ ist vorzugsweise Wasserstoff, Hydroxy, Fluor, Chlor, Brom, Cyano, Nitro, Methoxy, Ethoxy, Difluormethyl, Trifluormethyl, Chlormethyl, Trichlormethyl, 2,2,2-Trifluorethyl oder Pentafluorethyl,

$R^2$ ist vorzugsweise Wasserstoff, Fluor, Chlor, Hydroxy, Propyl, Ethyl, Methyl, Methoxy, Ethoxy, Difluormethyl, Trifluormethyl, Difluormethoxy, Trifluormethoxy, Methoxycarbonyl oder Ethoxycarbonyl,

$R^3$ ist vorzugweise Wasserstoff, Hydroxy, Fluor, Chlor, Brom, Cyano, Nitro, Difluormethyl, Trifluormethyl, Chlormethyl, Trichlormethyl, 2,2,2-Trifluorethyl, Pentafluorethyl, Difluormethoxy, Trifluormethoxy, Methylsulfonyl oder Ethylsulfonyl;

Q ist vorzugsweise der Rest

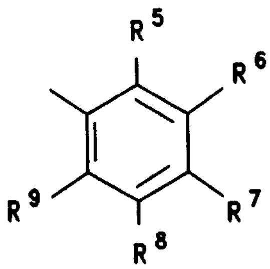 , 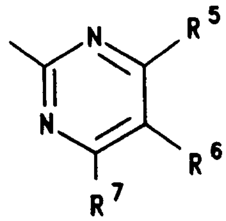 , 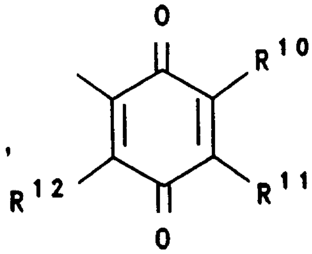

(Q1) (Q2) (Q3)

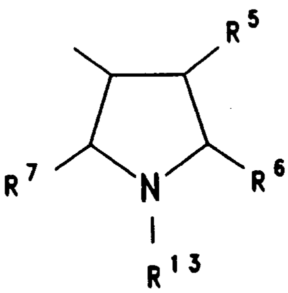

oder

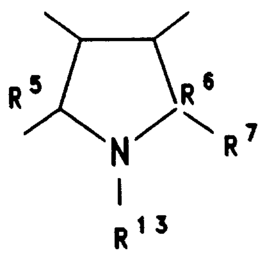

(Q4) (Q5)

wobei

$R^5$, $R^6$, $R^7$, $R^8$ und $R^9$ vorzugsweise und unabhängig voneinander Wasserstoff, Fluor, Chlor, Brom, Cyano, Nitro, Hydroxy, Hydroxysulfonyl, Ethoxysulfonyl, Ethylaminosulfonyl, Methylaminosulfonyl, Carboxyl, Ethoxycarbonyl, Methoxycarbonyl, Ethylaminocarbonyl, Methylaminocarbonyl, Ethoxycarbonyloxy, Methoxycarbonyloxy, Methylcarbonyloxy, t-Butyl, i-Propyl, Propyl, Ethyl, Methyl, Difluormethyl, Trifluormethyl, Chlormethyl, Trichlormethyl, Methoxy, Ethoxy, Difluormethoxy oder Trifluormethoxy,

$R^{10}$, $R^{11}$ und $R^{12}$ vorzugsweise und unbhängig voneinander Wasserstoff, Fluor, Chlor, Brom, Ethyl, Methyl, Difluormethyl, Trifluormethyl, Chlormethyl, Trichlormethyl, Methoxy, Ethoxy, Difluormethoxy oer Trifluormethoxy,

$R^{13}$ vorzugsweise Wasserstoff, Phenylsulfonyl, Methylsulfonyl, Ethylsulfonyl, Methylcarbonyl oder Ethylcarbonyl,

$R^{14}$ und $R^{15}$ vorzugsweise und unabhängig voneinander Wasserstoff, Butyl, Propyl, Ethyl, Methyl oder Benzyl bedeuten.

Bevorzugt sind auch erfindungsgemäße Verwendungen von Verbindungen der Formel (I), die eine Kombination der oben bevorzugten Merkmale aufweisen.

Die Verbindungen der genannten Formel (I) können beispielsweise hergestellt werden, indem man aromatische Carbonsäurechloride der Formel (II),

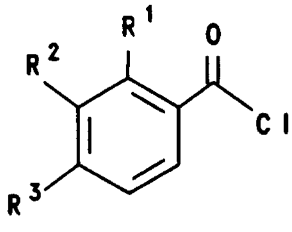

(II)

worin die Substituenten $R^1$, $R^2$ und $R^3$ die unter Formel (I) angegebenen Bedeutung haben, in Gegenwart einer Lewissäure in einem inerten Lösungmittel mit aromatischen oder heteroaromatischen Verbindungen

des Typs H-Q, worin Q die unter Formel (I) angegebene Bedeutung hat, umsetzt.

Die Herstellung der Verbindungen (I) erfolgt vorzugsweise in aprotischen Lösungsmitteln, insbesondere halogenierten Kohlenwasserstoffen wie z.B. Dichlormethan, Trichlormethan, 1,2-Dichlormethan oder 1,1,2,2-Tetrachlorethan bei -20 °C bis zum Siedepunkt des Reaktionsgemisches, insbesondere bei Raumtemperatur bis 50 °C, wobei die Umsetzung in Gegenwart einer katalytisch bis stöchiometrisch eingesetzten Menge Lewissäure wie z.B. Aluminiumchlorid, Titantetrachlorid oder Bortrifluorid erfolgt.

A. Herstellungsbeispiele

a) 3-(2,4-Dichlorbenzoyl)-1-phenylsulfonylpyrrol (Tabelle 1, Beispiel Nr. 150)

4.0 ml 2,4-Dichlorbenzoylchlorid werden mit 4.00 g Aluminiumchlorid in 50 ml 1,2-Dichlorethan 10 Minuten bei Raumtemperatur gerührt. Zu dieser Mischung tropft man eine Lösung aus 5.10 g Phenylsulfonylpyrrol in 20 ml 1,2-Dichlorethan und läßt 2 Stunden bei Raumtemperatur rühren. Die Mischung gießt man auf Eiswasser und extrahiert mit Dichlormethan. Die organische Phase wird getrocknet und eingedampft. Nach Umkristallisation des Rückstands aus einem Gemisch von Essigester und Petroether erhält man 8.3 g (89 % d.Th.) von 3-(2,4-Dichlor-benzoyl)-1-phenylsulfonylpyrrol als weiße Kristalle mit einem Schmelzpunkt von 122 °C.

b) 2,5'-Dichlor-2'-hydroxy-3'-methyl-4-methylsulfonyl-benzophenon (Tabelle 1, Beispiel Nr. 100)

Zu einer Mischung von 2.85 g 4-Chlor-2-methylphenol und 5.40 g Aluminiunchlorid in 100 ml 1,1,2,2-Tetrachlorethan gibt man bei Raumtemperatur portionsweise 5.06 g 2-Chlor-4-methylsulfonylbenzoylchlorid und erhitzt anschließend 7 Stunden unter Rückfluß. Nach dem Abkühlen gießt man das Reaktionsgemisch auf 200 g Eis und 500 ml konzentrierter Salzsäure. Die organische Phase wird abgetrennt und die wäßrige zweimal mit 50 ml Dichlormethan extrahiert. Die vereinigten organischen Phasen werden neutral gewaschen, über Magnesiumsulfat getrocknet und eingeengt. Nach Umkristallisieren aus einem Gemisch von Essigester und Diisopropylether erhält man 4.2 g (60 % d.Th.). 2,5'-Dichlor-2'-hydroxy-3'-methyl-4-methyl-sulfonylbenzophenon als farblose Kristalle mit einem Schmelzpunkt von 175-180 °C.

c) 2-(2,4-Dichlorbenzoyl)-1,4-chinon (Tabelle 1, Beispiel Nr. 91)

Eine Lösung von 2.49 g 2-(2,4-Dichlorbenzoyl)-1,4-dihydroxybenzol in 20 ml Eisessig wird bei 10 °C innerhalb von 3 Minuten zu einer Lösung von 1.60 g Natriumdichromat in 2 g konzentrierter Schwefelsäure und 60 ml Wasser getropft. Die Reaktionsmischung wird noch 30 Minuten bei 10-15 °C intensiv gerührt und anschließend abfiltriert. Der Rückstand wird mit Wasser säurefrei gewaschen und aus Diisopropylether umkristallisiert. Man erhält 1.74 g (70 % d.Th.) 2-(2,4-Dichlorbenzoyl)-1,4-chinon als gelborange Kristalle mit einem Schmelzpunkt von 100 °C.

d) 2,2',4'-Trichlor-6'-hydroxy-4-methylsulfonyl-benzophenon (Tabelle 1, Beispiel Nr. 183)

Zu einer Mischung von 1.97 g 3,5-Dichlorphenon und 3.23 g Aluminiumchlorid in 50 ml 1,1,2,2-Tetrachlorethan gibt man bei Raumtemperatur portionsweise 3.00 g 2-Chlor-4-methylsulfonyl-benzoylchlorid und erhitzt anschließend 7 Stunden unter Rückfluß. Nach dem Abkühlen gießt man das Reaktionsgemisch auf 100 g Eis und 20 ml konzentrierter Salzsäure. Die organische Phase wird abgetrennt und die wäßrige zweimal mit 50 ml Dichlormethan extrahiert. Die vereinigten organischen Phasen werden neutral gewaschen und anschließend dreimal mit je 25 ml 2n Natronlauge extrahiert. Die vereinigten alkalischen Extrakte werden mit konzentrierter Salzsäure auf einen pH-Wert von 2 gestellt. Der Niederschlag wird abfiltriert, mit Wasser neutral gewaschen und getrocknet. Man erhält 3.194 g (70 % d.Th.) 2,2',4'-Tri-chlor-6'-hydroxy-4-methylsulfonyl-benzophenon als farblose Kristalle mit einem Schmelzpunkt von 123-126 °C.

Die in Tabelle 1 aufgeführten Verbindungen werden analog zu den oben beschriebenen Herstellungsbeispielen erhalten.

Tabelle 1

Erfindungsgemäße Verbindungen der Formel (I)

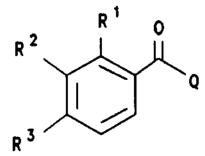

(I)

| Nr. | $R^1$ | $R^2$ | $R^3$ | Q | F.P. [°C] | $IC_{50}$ |
|---|---|---|---|---|---|---|
| 1 | Cl | H | $NO_2$ | 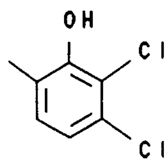 | 135-139 | $< 10^{-9}$ |
| 2 | Cl | $CH_3$ | $SO_2CH_3$ | 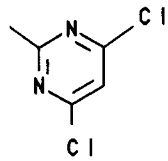 | | |
| 3 | Cl | H | $OCF_2H$ | 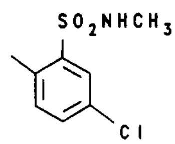 | | |

| | | | | | | |
|---|---|---|---|---|---|---|
| 4 | Br | H | CN | 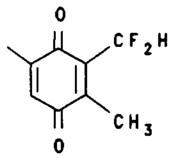 | | |
| 5 | Br | H | $NO_2$ | 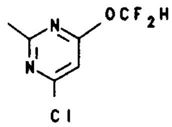 | | |
| 6 | Cl | $OC_2H_5$ | $SO_2CH_3$ | 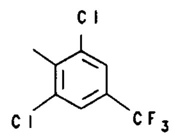 | | |
| 7 | Cl | H | $SO_2CH_3$ | 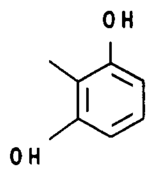 | 228 | $5.7x10^{-7}$ |
| 8 | F | OH | $CH_2CF_3$ | 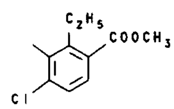 | | |

| | | | | | | |
|---|---|---|---|---|---|---|
| 9 | $NO_2$ | F | $CH_2Cl$ | 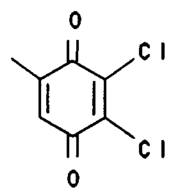 | | |
| 10 | Cl | H | $SO_2CH_3$ | 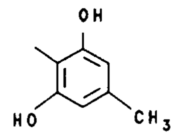 | 225 (Z.) | $1.8 \times 10^{-7}$ |
| 11 | $OC_2H_5$ | $CF_2H$ | $SO_2CH_3$ | 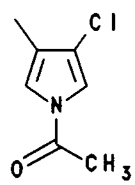 | | |
| 12 | $OCH_3$ | H | Br | 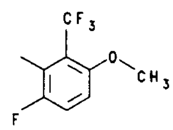 | | |
| 13 | OH | Cl | Cl | 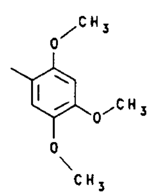 | 98-100 | $< 10^{-9}$ |

| | | | | | | |
|---|---|---|---|---|---|---|
| 14 | Cl | H | $SO_2CH_3$ | 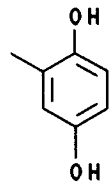 | 267 (Z.) | |
| 15 | F | $COOCH_3$ | $CH_2CF_3$ | 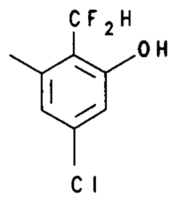 | | |
| 16 | Cl | H | Cl | 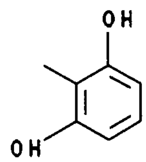 | 147 | |
| 17 | Cl | Cl | Br | 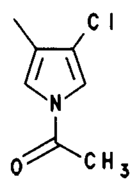 | | |
| 18 | CN | F | $CH_2Cl$ | 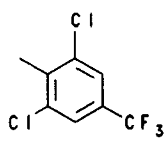 | | |

| | | | | | | |
|---|---|---|---|---|---|---|
| 19 | OH | Cl | Cl | 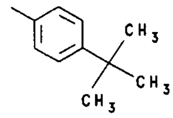 | 95-101 | |
| 20 | $CH_2Cl$ | $OCH_3$ | $NO_2$ | 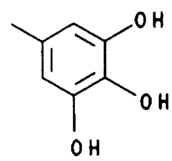 | | |
| 21 | Br | Cl | Cl | 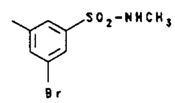 | | |
| 22 | OH | Cl | Cl | 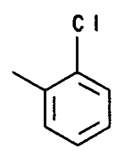 | 126-128 | |
| 23 | H | H | $SO_2CH_3$ | 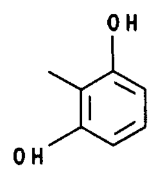 | 224 | |
| 24 | $CH_2CF_3$ | $COOCH_3$ | Cl | 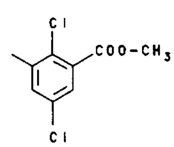 | | |

| | | | | | | |
|---|---|---|---|---|---|---|
| 25 | F | Cl | $SO_2CH_3$ | 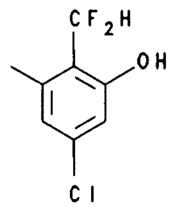 | | |
| 26 | OH | Cl | Cl | 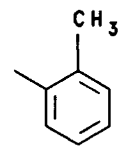 | 97-100 | |
| 27 | $NO_2$ | H | $CF_3$ | 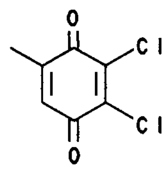 | | |
| 28 | Br | Cl | $OCF_2$ | 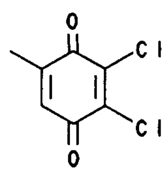 | | |
| 29 | Cl | H | $NO_2$ | 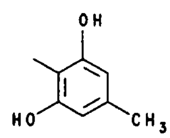 | 192 | |

| | | | | | | |
|---|---|---|---|---|---|---|
| 30 | $NO_2$ | H | Cl | 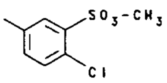 | | |
| 31 | $CCl_3$ | F | $SO_2CH_3$ | 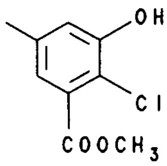 | | |
| 32 | Cl | H | $NO_2$ | 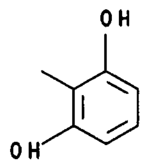 | 178 | |
| 33 | $NO_2$ | Cl | Cl | 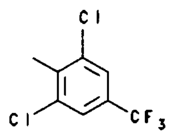 | | |
| 34 | OH | Cl | Cl | 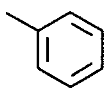 | 124-128 | |
| 35 | Cl | H | Cl | 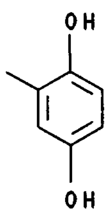 | 135 | < $x10^{-9}$ |

| | | | | | | |
|---|---|---|---|---|---|---|
| 36 | Cl | Cl | CN | 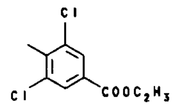 | | |
| 37 | Cl | H | $SO_2CH_3$ | 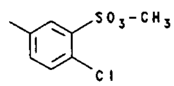 | | |
| 38 | Cl | H | $SO_2CH_3$ | 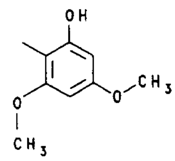 | 147 | |
| 39 | Cl | $COOCH_3$ | $SO_2CH_3$ | 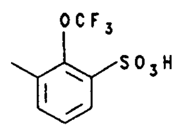 | | |
| 40 | Cl | H | Cl | 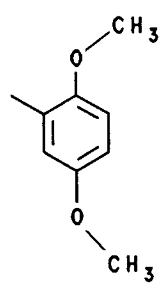 | 86 | $9.7x10^{-7}$ |

| | | | | | | |
|---|---|---|---|---|---|---|
| 41 | Cl | $C_3H_7$ | $SO_2CH_3$ | 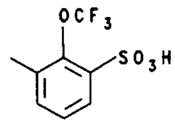 | | |
| 42 | OH | Cl | Cl | 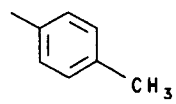 | 134-136 | $3.5x10^{-6}$ |
| 43 | Cl | H | Cl | 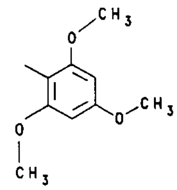 | 103 | $3.5x10^{-6}$ |
| 44 | H | $OCF_3$ | F | 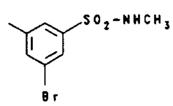 | | |
| 45 | $NO_2$ | H | Cl | 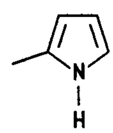 | 125 | |
| 46 | Cl | $CH_3$ | $CF_2H$ | 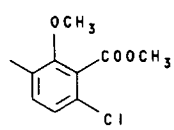 | | |

| | | | | | | |
|---|---|---|---|---|---|---|
| 47 | Cl | H | $SO_2CH_3$ | 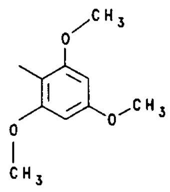 | 163-166 | $5.7x10^{-6}$ |
| 48 | F | $COOCH_3$ | $OCF_2H$ | 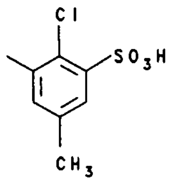 | | |
| 49 | Cl | H | Cl | 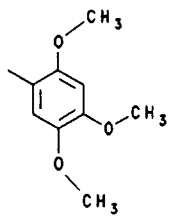 | 99 | |
| 50 | OH | Cl | Cl | 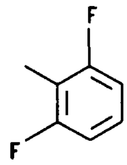 | 98-103 | |

| | | | | | | |
|---|---|---|---|---|---|---|
| 51 | $CH_2Cl$ | $C_3H_7$ | $SO_2CH_3$ | 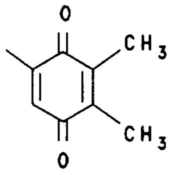 | | |
| 52 | $NO_2$ | OH | Br | 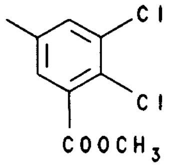 | | |
| 53 | $CF_3$ | H | Cl | 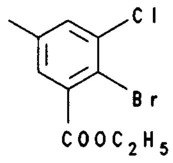 | | |
| 54 | Cl | H | Cl | 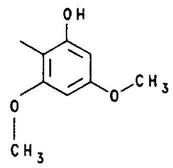 | | |
| 55 | Cl | Cl | Cl | 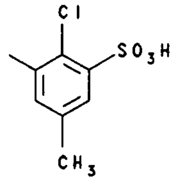 | | |

| | | | | | | |
|---|---|---|---|---|---|---|
| 56 | OH | Cl | Cl | 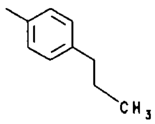 | 64-65 | $5.0x10^{-8}$ |
| 57 | Br | H | $OCF_3$ | 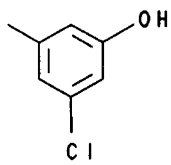 | | |
| 58 | F | $CH_3$ | F | 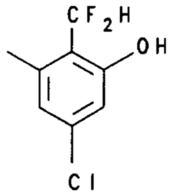 | | |
| 59 | Br | Cl | Cl | 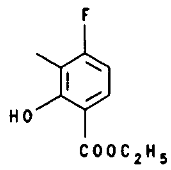 | | |
| 60 | Cl | H | $SO_2CH_3$ | 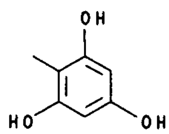 | 165 | |

| | | | | | | |
|---|---|---|---|---|---|---|
| 61 | $NO_2$ | H | $OCF_2H$ | 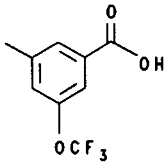 | | |
| 62 | $CF_3$ | Cl | Cl | 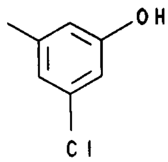 | | |
| 63 | Cl | H | $SO_2CH_3$ | 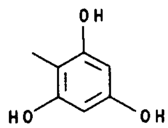 | 227 | |
| 64 | $CCl_3$ | H | Cl | 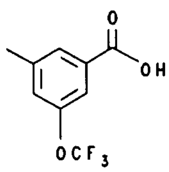 | | |
| 65 | Cl | H | $CF_3$ | 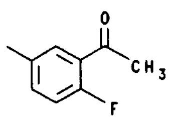 | | |

| | | | | | | |
|---|---|---|---|---|---|---|
| 66 | Cl | H | $SO_2CH_3$ | 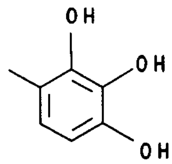 | 228 | |
| 67 | Cl | $CH_3$ | $OCF_2H$ | 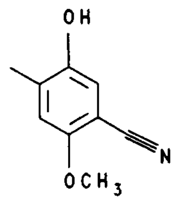 | | |
| 68 | Cl | $CF_3$ | $OCF_2H$ | 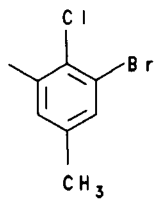 | | |
| 69 | Cl | H | $SO_2CH_3$ | 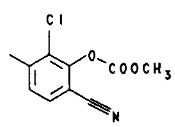 | | |
| 70 | Cl | H | $SO_2CH_3$ | 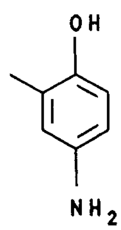 | 240 (Z.) | $4.0x10^{-8}$ |

| | | | | | | |
|---|---|---|---|---|---|---|
| 71 | $OCH_3$ | $COOCH_3$ | $SO_2CH_3$ | 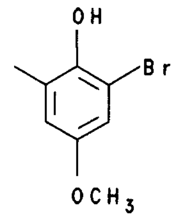 | | |
| 72 | Cl | H | $SO_2C_2H_5$ | 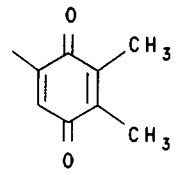 | | |
| 73 | $CF_3$ | $CH_3$ | Cl | 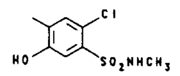 | | |
| 74 | Cl | H | $SO_2CH_3$ | 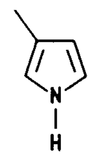 | 151-153 | |
| 75 | F | $OCF_3$ | Cl | 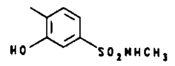 | | |
| 76 | Cl | $CF_3$ | Cl | 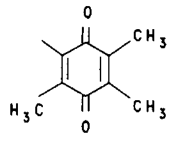 | | |

| | | | | | | |
|---|---|---|---|---|---|---|
| 77 | H | $COOC_2H_5$ | $SO_2CH_3$ | 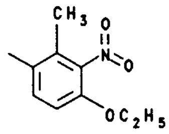 | | |
| 78 | Cl | H | $SO_2CH_3$ | 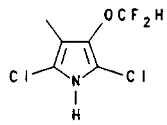 | | |
| 79 | $NO_2$ | H | Cl | 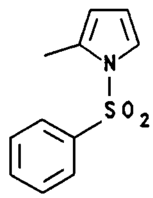 | 160-162 | |
| 80 | F | Cl | Cl | 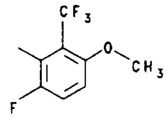 | | |
| 81 | $OCH_3$ | $COOCH_3$ | $SO_2CH_3$ | 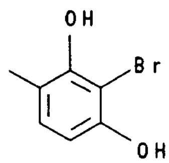 | | |

| | | | | | | |
|---|---|---|---|---|---|---|
| 82 | Cl | Cl | OH | 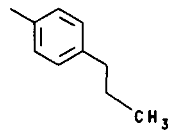 | 137-139 | |
| 83 | Cl | Cl | $CF_3$ | 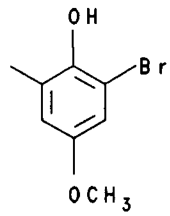 | | |
| 84 | F | $C_2H_5$ | Cl | 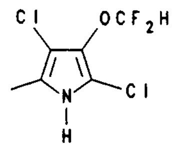 | | |
| 85 | $OCF_2H$ | Cl | Cl | 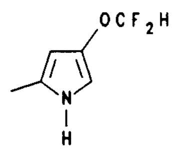 | | |
| 86 | Cl | H | $SO_2CH_3$ | 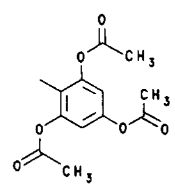 | 157 | |

| | | | | | | |
|---|---|---|---|---|---|---|
| 87 | Cl | H | $SO_2CH_3$ | 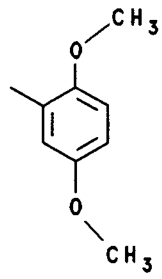 | 108 | $2.4x10^{-7}$ |
| 88 | Cl | H | $SO_2CH_3$ | 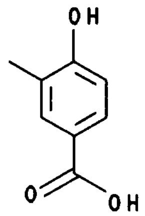 | 254-256 | $7.0x10^{-8}$ |
| 89 | $CF_2H$ | Cl | Cl | 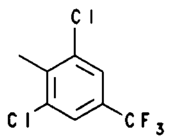 | | |
| 90 | Br | H | $SO_2$ | 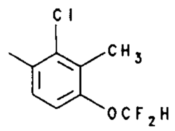 | | |
| 91 | Cl | H | Cl | 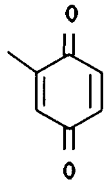 | 100 | $1.8x10^{-8}$ |

| | | | | | | |
|---|---|---|---|---|---|---|
| 92 | Cl | $C_2H_5$ | $CF_3$ | 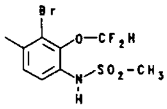 | | |
| 93 | F | F | F | 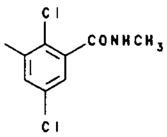 | | |
| 94 | Cl | Cl | OH | 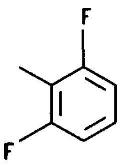 | 149-152 | |
| 95 | OH | Cl | Cl | 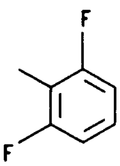 | 118-121 | |
| 96 | $OC_2H_5$ | Cl | $CF_3$ | 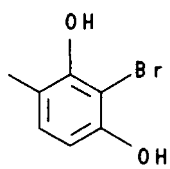 | | |

| | | | | | | |
|---|---|---|---|---|---|---|
| 97 | Cl | H | $SO_2CH_3$ | 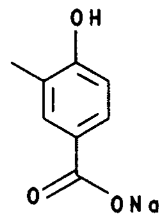 | 180 (Z.) | $6.0x10^{-8}$ |
| 98 | CN | F | F | 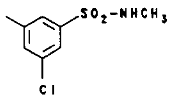 | | |
| 99 | Cl | H | $SO_2CH_3$ | 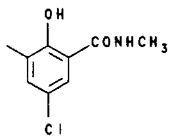 | | |
| 100 | Cl | H | $SO_2CH_3$ | 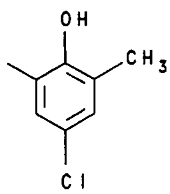 | 175-180 | $6.2x10^{-9}$ |
| 101 | Cl | H | $SO_2CH_3$ | 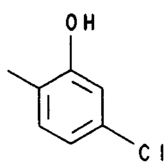 | 183 | $< 10^{-9}$ |

| | | | | | | |
|---|---|---|---|---|---|---|
| 102 | Cl | $OCH_3$ | $NO_2$ | 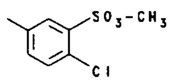 | | |
| 103 | F | Cl | Cl | 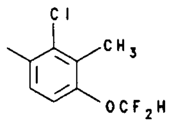 | | |
| 104 | Cl | H | Cl | 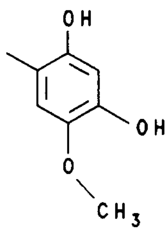 | 90 | |
| 105 | Cl | H | $SO_2C_2H_5$ | 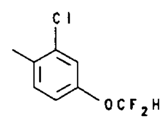 | | |
| 106 | Cl | H | $SO_2CH_3$ | 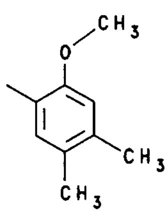 | 141-143 | |

| | | | | | | |
|---|---|---|---|---|---|---|
| 107 | H | $COOC_2H_5$ | CN | 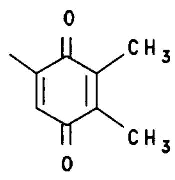 | | |
| 108 | $OCH_3$ | H | $CF_3$ | 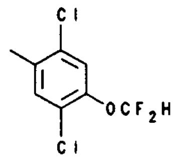 | | |
| 109 | Cl | H | $SO_2CH_3$ | 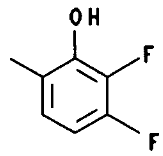 | 171-175 | $< 10^{-8}$ |
| 110 | H | $COOC_2H_5$ | $CF_3$ | 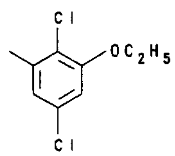 | | |
| 111 | $CCl_3$ | Cl | Cl | 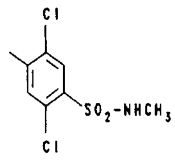 | | |

| | | | | | | |
|---|---|---|---|---|---|---|
| 112 | $CCl_3$ | Cl | Cl | 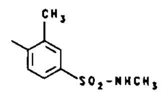 | | |
| 113 | $CF_3$ | $OCH_3$ | Cl | 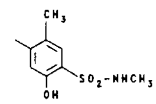 | | |
| 114 | Cl | H | $SO_2CH_3$ | 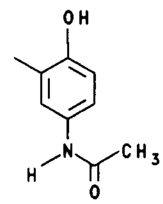 | 234-235 | $9.0x10^{-9}$ |
| 115 | Cl | H | $SO_2CH_3$ | 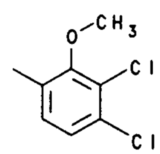 | 98-100 | |
| 116 | Cl | Cl | Br | 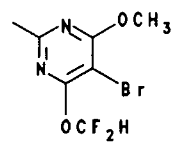 | | |
| 117 | CN | H | Cl | 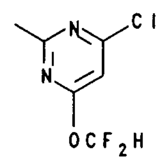 | | |

| | | | | | | |
|---|---|---|---|---|---|---|
| 118 | Cl | $CF_3$ | Cl | 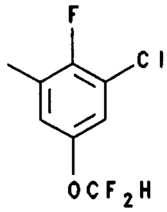 | | |
| 119 | $NO_2$ | $OC_2H_5$ | Cl | 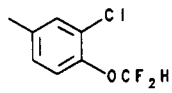 | | |
| 120 | Cl | H | $SO_2CH_3$ | 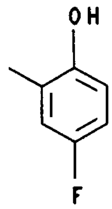 | 182-187 | $< 10^{-9}$ |
| 121 | F | $OCH_3$ | F | 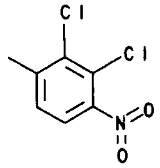 | | |
| 122 | Cl | Cl | Cl | 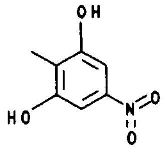 | | |
| 123 | $CF_3$ | $COOCH_3$ | Cl | 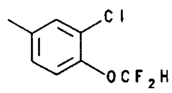 | | |

| | | | | | | |
|---|---|---|---|---|---|---|
| 124 | Cl | H | $SO_2CH_3$ | 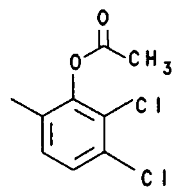 | 188-192 | |
| 125 | Cl | $COOC_2H_5$ | Cl | 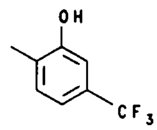 | | |
| 126 | $NO_2$ | H | Cl | 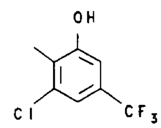 | | |
| 127 | Cl | H | $SO_2CH_3$ | 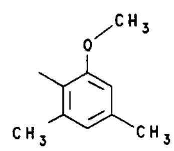 | 136-140 | |
| 128 | OH | $CF_2H$ | Br | 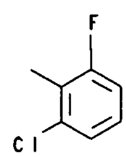 | | |
| 129 | F | $CH_3$ | $SO_2CH_3$ | 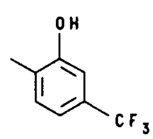 | | |

| | | | | | | |
|---|---|---|---|---|---|---|
| 130 | Cl | H | $SO_2CH_3$ | 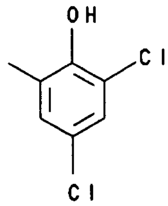 | 198-202 | $1.0x10^{-9}$ |
| 131 | Cl | H | $CF_3$ | 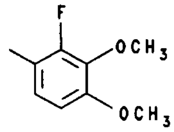 | | |
| 132 | Cl | H | Cl | 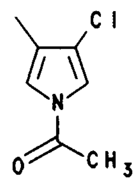 | | |
| 133 | H | Cl | Cl | 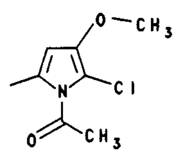 | | |
| 134 | Cl | H | $SO_2CH_3$ | 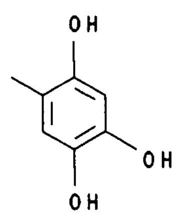 | 278 | |

| | | | | | | |
|---|---|---|---|---|---|---|
| 135 | Cl | $OC_2H_5$ | $C_2F_5$ | 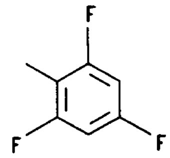 | | |
| 136 | Cl | H | $SO_2CH_3$ | 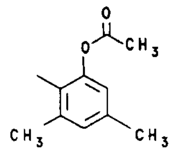 | 105-108 | $3.0x10^{-7}$ |
| 137 | $C_2F_5$ | $COOCH_3$ | Cl | 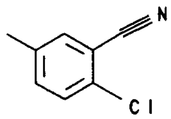 | | |
| 138 | Cl | H | Cl | 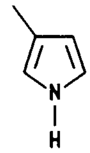 | 173 | |
| 139 | CN | F | Cl | 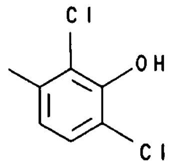 | | |
| 140 | H | Cl | $SO_2CH_3$ | 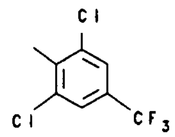 | | |

| | | | | | | |
|---|---|---|---|---|---|---|
| 141 | Cl | H | $SO_2CH_3$ | 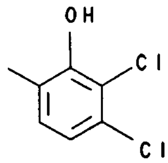 | 182-185 | $1.1x10^{-9}$ |
| 142 | Cl | $CF_2H$ | $SO_2C_2H_5$ | 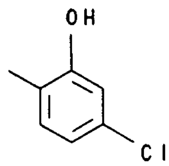 | | |
| 143 | $OC_2H_5$ | $COOCH_3$ | $SO_2CH_3$ | 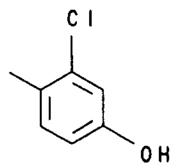 | | |
| 144 | $CF_3$ | Cl | $SO_2CH_3$ | 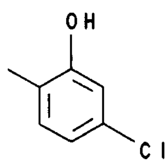 | | |
| 145 | $NO_2$ | Cl | $OCF_2H$ | 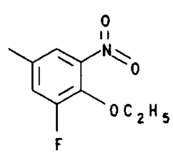 | | |

| | | | | | | |
|---|---|---|---|---|---|---|
| 146 | Cl | H | Cl | 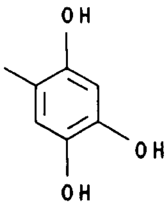 | 178 | |
| 147 | $CH_2Cl$ | Cl | $SO_2CH_3$ | 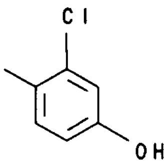 | | |
| 148 | Cl | H | $SO_2CH_3$ | 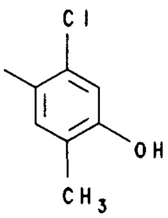 | 193-195 | $9.2x10^{-7}$ |
| 149 | $CF_2H$ | H | $OCF_3$ | 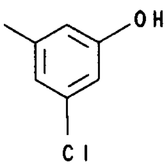 | | |

| | | | | | | |
|---|---|---|---|---|---|---|
| 150 | Cl | H | Cl | 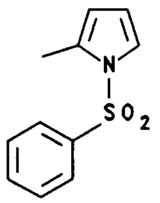 | 122 | |
| 151 | F | $CF_3$ | F | 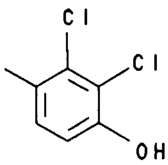 | | |
| 152 | Cl | H | $SO_2CH_3$ | 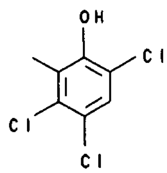 | 143-144 | $1.2x10^{-7}$ |
| 153 | Cl | Cl | Cl | 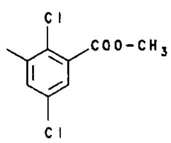 | | |
| 154 | Cl | $COOCH_3$ | $CF_3$ | 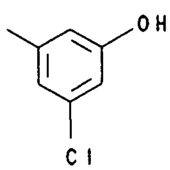 | | |

| | | | | | | |
|---|---|---|---|---|---|---|
| 155 | $C_2F_5$ | H | $NO_2$ | 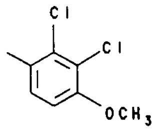 | | |
| 156 | Cl | H | $SO_2CH_3$ | 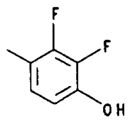 | 160-165 | $< 10^{-9}$ |
| 157 | $CF_3$ | H | F | 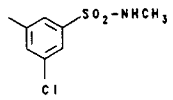 | | |
| 158 | Cl | $CF_2H$ | $SO_2C_2H_5$ | 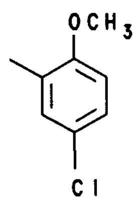 | | |
| 159 | $C_2F_5$ | H | Cl | 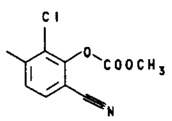 | | |
| 160 | Cl | H | $SO_2CH_3$ | 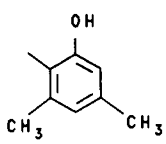 | 142-145 | $2.3x10^{-7}$ |

| | | | | | | |
|---|---|---|---|---|---|---|
| 161 | $CCl_3$ | H | $SO_2CH_3$ | 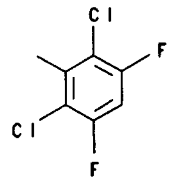 | | |
| 162 | Cl | $CF_3$ | Cl | 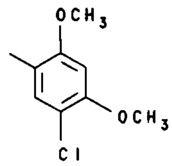 | | |
| 163 | Cl | H | $SO_2CH_3$ | 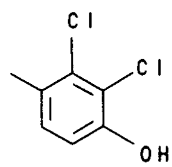 | 167-170 | $1.2x10^{-8}$ |
| 164 | Br | $OCF_2H$ | $CF_3$ | 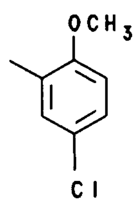 | | |
| 165 | Cl | $OCH_3$ | $SO_2CH_3$ | 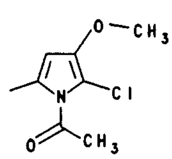 | | |

| | | | | | | |
|---|---|---|---|---|---|---|
| 166 | Cl | H | $SO_2CH_3$ | 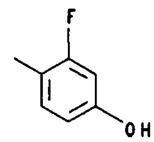 | 154-155 | $2.0x10^{-8}$ |
| 167 | H | F | $CCl_3$ | 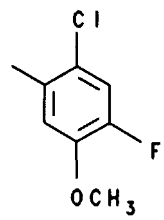 | | |
| 168 | Cl | H | $SO_2CH_3$ | 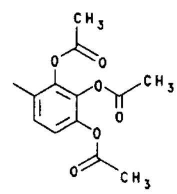 | 161 | |
| 169 | $CH_2CF_3$ | Cl | Cl | 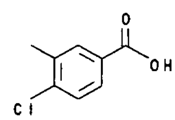 | | |
| 170 | $CF_2H$ | $OCH_3$ | CN | 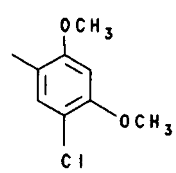 | | |

| | | | | | | |
|---|---|---|---|---|---|---|
| 171 | Cl | H | $SO_2CH_3$ | 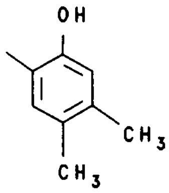 | 228-230 | $2.2x10^{-8}$ |
| 172 | $CF_3$ | H | Cl | 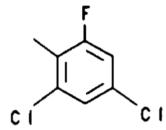 | | |
| 173 | Cl | H | Cl | 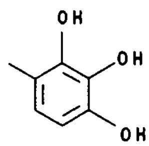 | 92 | |
| 174 | Br | $OCF_3$ | $CH_2Cl$ | 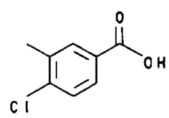 | | |
| 175 | Cl | H | $SO_2CH_3$ | 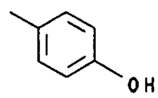 | 195-196 | $1.2x10^{-8}$ |
| 176 | OH | $COOC_2H_5$ | $CF_2H$ | 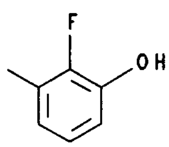 | | |

| | | | | | | |
|---|---|---|---|---|---|---|
| 177 | H | $OCH_3$ | F | 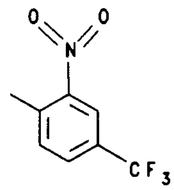 | | |
| 178 | Cl | H | $SO_2CH_3$ | 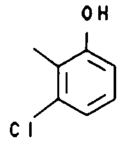 | 118-120 | $4.0x10^{-9}$ |
| 179 | Br | $OCH_3$ | $CF_3$ | 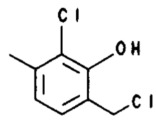 | | |
| 180 | $NO_2$ | $COOCH_3$ | $OCF_2H$ | 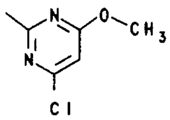 | | |
| 181 | Cl | H | Cl | 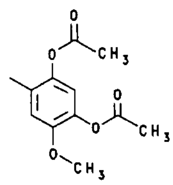 | 134 | |
| 182 | CN | F | $CH_2CF_3$ | 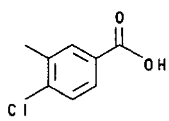 | | |

| | | | | | | |
|---|---|---|---|---|---|---|
| 183 | Cl | H | $SO_2CH_3$ | 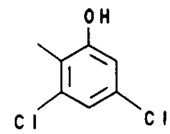 | 123-125 | $4.8x10^{-9}$ |
| 184 | Br | $OCH_3$ | $CF_2H$ | 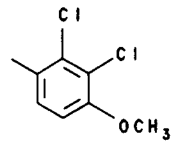 | | |
| 185 | Cl | $OCF_2H$ | $NO_2$ | 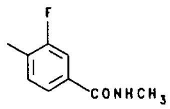 | | |
| 186 | $OC_2H_5$ | $COOC_2H_5$ | Cl | 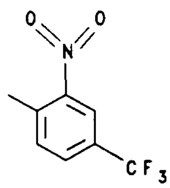 | | |
| 187 | Cl | H | $SO_2CH_3$ | 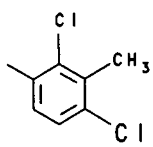 | 117-121 | $4.0x10^{-7}$ |
| 188 | F | Cl | $CCl_3$ | 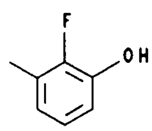 | | |

| | | | | | | |
|---|---|---|---|---|---|---|
| 189 | Cl | H | Cl | 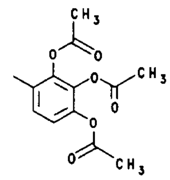 | 101 | |
| 190 | Cl | $OCF_3$ | $SO_2CH_3$ | 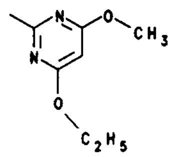 | | |
| 191 | Cl | H | $SO_2CH_3$ | 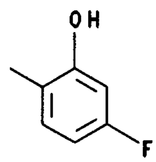 | 164-166 | $< 10^{-8}$ |
| 192 | Br | OH | $CF_3$ | 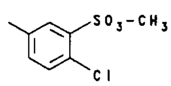 | | |
| 193 | OH | Cl | Cl | 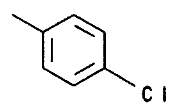 | 116-118 | |
| 194 | Cl | Cl | OH | 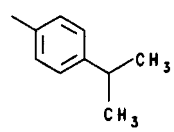 | 69-71 | |

| | | | | | | |
|---|---|---|---|---|---|---|
| 195 | Br | H | $CCl_3$ | 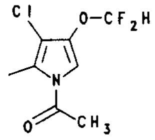 | | |
| 196 | Cl | H | $SO_2CH_3$ | 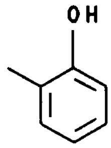 | 162-168 | $< 10^{-9}$ |
| 197 | F | H | Br | 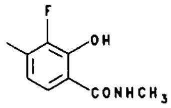 | | |
| 198 | Cl | H | Cl | 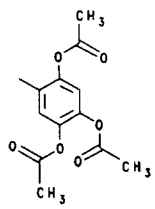 | 132 | |
| 199 | Cl | Br | $CF_3$ | 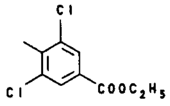 | | |
| 200 | Cl | H | Cl | 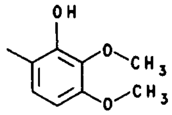 | 135 | |

| | | | | | | |
|---|---|---|---|---|---|---|
| 201 | Cl | H | $SO_2CH_3$ | 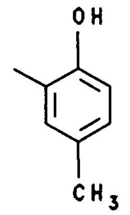 | 115-118 | < $10^{-9}$ |
| 202 | F | $COOCH_3$ | $SO_2CH_3$ | 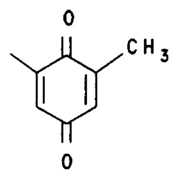 | | |
| 203 | $CH_2CF_3$ | H | Br | 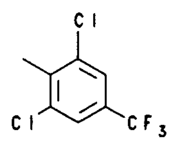 | | |
| 204 | Cl | H | $SO_2CH_3$ | 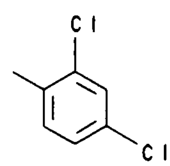 | 118-121 | < $10^{-5}$ |

Die Erfindung betrifft ferner den Einsatz der mit dem erfindungsgemäßen Testverfahren identifizierten herbiziden Wirkstoffe in Pflanzenpopulationen.

Als Pflanzenpopulationen kommen insbesondere Kulturpflanzen in Frage.

In den folgenden Beispielen wird die Erfindung weitergehend beschrieben und anhand von Beispielen näher erläutert. Prozentangaben beziehen sich auf das Gewicht, wenn nichts anderes angegeben wird.

Beispiel 1: Anreicherung der HPPD aus Maispflanzen

Zur Anreicherung der anabolen HPPD aus pflanzlichen Geweben wurden junge etiolierte Maispflanzen mit Extraktionspuffer (20 mM Phosphatpuffer pH 7,0; 0,14 M KCl; 0,1 mg/ml Glutathion und 1 % unlösliches Polyvinylpyrolidon) für 3x10 Sekunden homogenisiert, z.B. in einem Waring blendor. Der Extrakt wurde filtriert (alle weiteren Isolationsschritte wurden bei 4 °C durchgeführt) und zentrifugiert (20 min; 10.000 g). Der Überstand wurde einer fraktionierten Ammoniumsulfatfällung unterzogen. Die HPPD fiel bei einer Sättigung der Lösung mit Ammoniumsulfat zwischen 20 und 40% aus. Aus dieser Stufe konnte das Enzym nach eine Dialyse in einem geeigneten Puffer, wie z. B. 20 mM Phosphatpuffer pH 7,0; 0,14 M KCl; 0,1 mg/ml Glutathion, bei -80 °C gelagert werden. Dieser Reinheitsgrad des Enzyms reicht zur Identifizierung von Inhibitoren aus.

Beispiel 2: Verfahren zur Identifizierung potentieller Hemmstoffe der HPPD

Die Enzymtests wurden in 20 ml Schraubgefäßen aus Polyethylen durchgeführt. Zwei Injektionskanülen (0.9 x 40 mm) wurden durch den Schraubdeckel gesteckt. Eine der Kanülen wurde zu einem Haken gebogen, an den ein Filterpapierscheibchen mit 10 mm Durchmesser gehängt wurde. Das Filterpapier wurde mit 25 µl Methylbenzethonium-Hydroxyd (Sigma) befeuchtet. Die zweite Nadel diente der Injektion von Start- und Stoplösung in das Schraubgefäß.

Der Enzym-Reaktionsmix enthielt:

1 ml 0.1 M Phosphatpuffer, pH 7,3;

2 g/l Rinderleber Katalase;

100 µl von einer Mischung aus 3 mM Dichlorphenolindophenol und 150 mM Glutathion (reduziert);

25 µl Aceton (oder potentielle Hemmstoffe, gelöst in Aceton) und

Enzym in einem Gesamtvolumen von 1635 µl.

Nach 5minütiger Vorinkubation bei 30 °C wurde die Reaktion durch Injektion von 800 µl $^{14}C$-p-Hydroxyphenylpyruvat gestartet. Durch Injektion von 600 µl 1N $H_2SO_4$ wurde die Reaktion gestoppt, weitere 15 Minuten inkubiert und die freigesetzte Radioaktivität auf den Filterblättchen im Flüssigkeits-Szintillationszähler ermittelt.

Beispiel 3: Nachweis der Hemmung der Plastochinonbiosynthese in Weizen durch SC-0051

Dünnschichtchromatographische Analyse der Inkorporation von Radioaktivität aus $^{14}C$-Tyrosin in Plastochinon. Weizenpflanzen wurden entweder nicht behandelt (A), mit einer Standard-Screening-Formulierung (B) oder mit 100 g/ha SC-0051 in dieser Screening-Formulierung gesprüht (C) und dann mit $^{14}C$-Tyrosin inkubiert. Die lipophilen Metabolite des Weizens wurden mit Chloroform extrahiert, getrennt auf Silica-Gel-60-HPTLC-Platten mit Chloroform:

Diethylether (99:1) und die Radioaktivität in mittels Dünnschichtscanner analysiert. Man erkennt deutlich den Einbau von Radioaktivität aus Tyrosin in Plastochinon ( siehe Abbildung 1, Bande 2) in den Kontrollansätzen (A) und (B), wohingegen die Plastochinonbiosynthese durch den Einsatz von SC-0051 gehemmt ist (C).

Beispiel 4: Hemmung der partiell gereinigten Mais HPPD durch das Herbizid SC-0051.

Analog zu Beispiel 3 wurde der Test mit einem anderen Hemmstoff (Clomazone) durchgeführt, der auch zu Bleichungserscheinungen führt. Während SC-5001 die HPPD deutlich hemmt (○), hat das andere bleichende Herbizid keine inhibierende Wirkung auf die HPPD (●) (siehe Abbildung 2).

Beispiel 5: Nachweis, daß die Hemmung der HPPD mit herbizider Wirkung korreliert ist

HPPD katalysiert die Transformation von p-Hydroxyphenylpyruvat zu Homogentisinsäure. Wenn die Hemmung der HPPD für die herbizide Wirkung eines Hemmstoffes verantwortlich wäre, so müßte sich der herbizide Effekt durch eine Supplementation mit Homogentisinsäure aufheben lassen. Dieser Nachweis konnte durch einen Versuch an der Wasserlinse Lemna gibba erbracht werden. Die Pflanze wurden in einem sterilem wäßrigem Medium mit den Zusätzen wie in Tabelle 2 beschrieben kultiviert.

Tabelle 2

| Aufhebung der Wirkung von SC-0051 durch Homogentisinsäure | | |
|---|---|---|
| Medienzusatz | Anzahl der L.gibba Pflanzen | |
| | Kontrolle | $10^{-7}$ M SC-0051 |
| - | 25 | 3 |
| 1 mM HPPD | 22 | 2 |
| 1 mM HGA | 29 | 32 |

Jeweils drei Lemna Pflanzen wurden in 100 ml sterilem Medium sechs Tage lang mit sterilfiltierten Zusätzen inkubiert. SC-0051 wurde in Form einer $10^{-3}$M Stammlösung (Wirkstoff in Aceton) zugegeben. Die Anzahl der neu entstandenen Pflanzen wurde am sechsten Tag bestimmt.

Beispiel 6: Wirkung von Benzophenonen

| Beispiel Nr. aus Tabelle I | $IC_{50}$-Wert (M) | Lemna Wachstumshemmung (%) | | |
|---|---|---|---|---|
| | | $10^{-4}$ M | $10^{-5}$ M | $10^{-6}$ M |
| 183 | $4.8 \times 10^{-9}$ | 98 | 82 | 81 |
| 160 | $2.3 \times 10^{-7}$ | 80 | 72 | 12 |
| 204 | $> 10^{-5}$ | 28 | 12 | -- |
| 171 | $2.2 \times 10^{-8}$ | 98 | 93 | 93 |
| 100 | $6.2 \times 10^{-9}$ | 98 | 96 | 92 |

Tabelle 2 zeigt die $IC_{50}$-Werte der Hemmung der p-Hydroxyphenylpyruvat Dioxygenase durch verschiedene Benzophenonderivate verglichen mit der Hemmung des Wachstums von Lemna gibba.

Der $IC_{50}$-Wert ist die molare Konzentration des Hemmstoffs, bei der 50 % der Enzymaktivität gehemmt sind. Das Wachstum von Lemna wurde gemessen wie beschrieben in: Schulz, A., Ort, O., Beyer, P. und Kleinig, H.; SC-0051, a 2-benzoyl-cyclohexane-1,3-dione bleaching herbicide, is a potent inhibitor of the enzyme p-hydroxyphenylpyruvate dioxygenase. FEBS-Letters, 1993, 318 (2), 162-166.

Aus der Tabelle ist ersichtlich, daß die herbizide Wirkung der Benzophenonderivate mit der in vitro gemessenen Aktivität korreliert.

**Patentansprüche**

1. Ein Verfahren zur Anreicherung der anabolen p-Hydroxyphenylpyruvat-Dioxygenase aus Pflanzenzellen, dadurch gekennzeichnet, daß man das Enzym direkt isoliert.

2. Ein Verfahren gemäß Anspruch 1, dadurch gekennzeichnet, daß man pflanzliche Gewebe oder Zellen mit Extraktionspuffer homogenisiert, den Extrakt filtriert, den Überstand einer fraktionierten Ammoniumsulfatfällung unterzieht und das entstandene Präzipitat wieder in einem geeigneten Puffer löst.

3. Eine gereinigte anabole p-Hydroxyphenylpyruvat-Dioxygenase aus Pflanzen, erhältlich nach einem Verfahren gemäß Anspruch 1.

4. Ein Testsystem zur Identifizierung von Inhibitoren der anabolen p-Hydroxyphenylpyruvat-Dioxygenase aus Pflanzen, dadurch gekennzeichnet, daß man das Enzym mit einem zu untersuchenden Testsubstrat inkubiert und nach einer geeigneten Reaktionszeit die enzymatische Aktivität des Enzyms im Vergleich zur Aktivität des nicht gehemmten Enzyms ermittelt.

5. Ein Inhibitor der p-Hydroxyphenylpyruvat-Dioxygenase, der nach einem Verfahren gemäß Anspruch 4 identifiziert wurde.

6. Ein herbizides Mittel, das einen Hemmstoff der p-Hydroxyphenylpyruvat-Dioxygenaseenthält, der nach einem Verfahren gemäß Anspruch 4 identifiziert wurde.

7. Einsatz der herbiziden Mittel gemäß Anspruch 6 in Pflanzenpopulationen.

8. Verbindungen der Formel (I), worin
   - $R^1$ Wasserstoff, Halogen, OH, Alkoxy, CN, $NO_2$ oder Halogenalkyl,
   - $R^2$ Wasserstoff, Halogen, OH, Alkyl, Alkoxy, Halogenalkyl, Halogenalkoxy oder Alkoxycarbonyl,
   - $R^3$ Wasserstoff, Halogen, OH, CN, $NO_2$, Halogenalkyl, Halogenalkoxy oder $R^4S(O)_m$-, wobei $R^4$ Alkyl und m null, eins oder zwei sind,
   - Q einen Rest aus der Gruppe der Formel

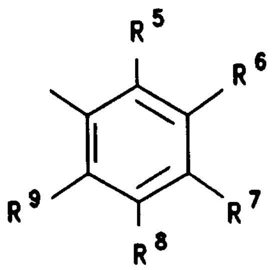,
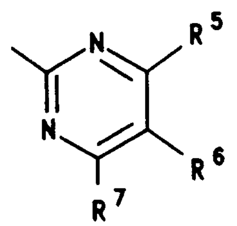,
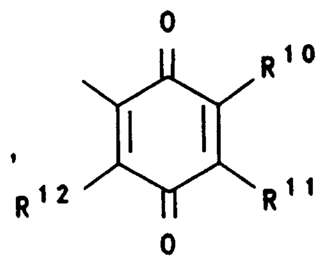

(Q1) (Q2) (Q3)

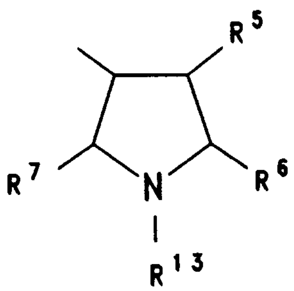

oder

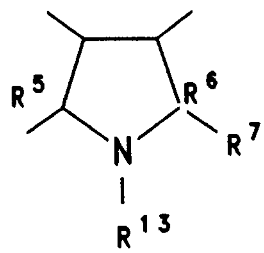

(Q4) (Q5)

$R^5$, $R^6$, $R^7$, $R^8$ und $R^9$ unabhängig voneinander Wasserstoff, Halogen, OH, CN, $NO_2$, $SO_3H$, $SO_3R^4$, $SO_2NR^{14}R^{15}$, COOH, $COOR^4$, $CONR^{14}R^{15}$, O-$COOR^4$, O-$COR^4$, Alkyl, Alkoxy, Halogenalkyl oder Halogenalkoxy,

$R^{10}$, $R^{11}$ und $R^{12}$ unabhängig voneinander Wasserstoff, Halogen, Alkyl, Alkoxy, Halogenalkyl oder Halogenalkoxy,

$R^{13}$ Wasserstoff, Phenylsulfonyl, Alkylsulfonyl oder Alkylcarbonyl,

$R^{14}$ und $R^{15}$ unabhängig voneinander Wasserstoff, Alkyl, Aryl oder Benzyl bedeuten, als Herbizide.

**9.** Verbindungen der Formel (I) nach Anspruch 8, dadurch gekennzeichnet, daß

- $R^1$ Wasserstoff, Halogen, OH, ($C_1$-$C_3$)-Alkoxy, CN, $NO_2$ oder ($C_1$-$C_3$)-Halogenalkyl,
- $R^2$ Wasserstoff, Halogen, OH, ($C_1$-$C_3$)-Alkyl, ($C_1$-$C_3$)-Alkoxy, ($C_1$-$C_2$)-Halogenalkyl, ($C_1$-$C_2$)-Halogenalkoxy oder ($C_1$-$C_2$-Alkoxy)-carbonyl,
- $R^3$ Wasserstoff, Halogen, OH, CN, $NO_2$, ($C_1$-$C_3$)-Halogenalkyl, ($C_1$-$C_2$)-Halogenalkoxy oder $R^4S$-$(O)_m$-, wobei $R^4$ ($C_1$-$C_2$)-Alkyl und m null, eins oder zwei sind,
- Q einen Rest aus der Gruppe der Formel

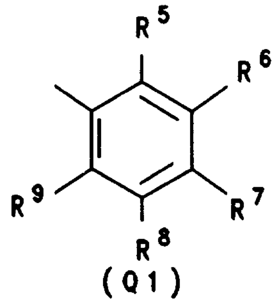
(Q1)
,
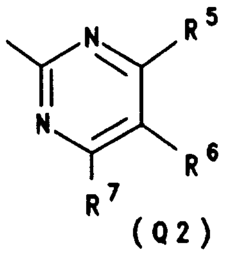
(Q2)
,
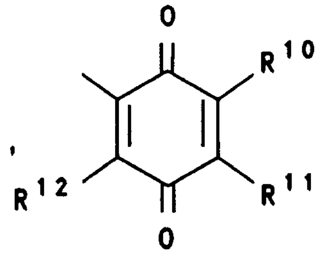
(Q3)

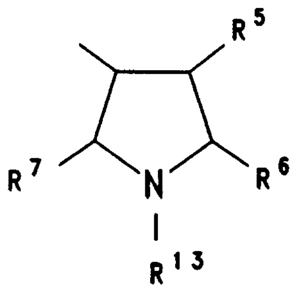
(Q4)

oder

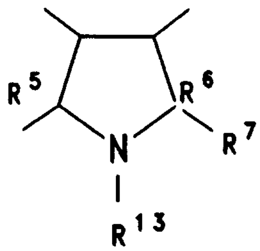
(Q5)

$R^5$, $R^6$, $R^7$, $R^8$ und $R^9$ unabhängig voneinander Wasserstoff, Halogen, OH, CN, $NO_2$, $SO_3H$, $SO_3R^4$, $SO_2NR^{14}R^{15}$, COOH, $COOR^4$, $CONR^{14}R^{15}$, O-$COOR^4$, O-$COR^4$, ($C_1$-$C_4$)-Alkyl, ($C_1$-$C_2$)-Alkoxy, ($C_1$-$C_3$)-Halogenalkyl oder ($C_1$-$C_2$)-Halogenalkoxy,
$R^{10}$, $R^{11}$ und $R^{12}$ unabhängig voneinander Wasserstoff, Halogen, ($C_1$-$C_3$)-Alkyl, ($C_1$-$C_2$)-Alkoxy, ($C_1$-$C_3$)-Halogenalkyl oder ($C_1$-$C_2$)-Halogenalkoxy,
$R^{13}$ Wasserstoff, Phenylsulfonyl, ($C_1$-$C_2$)-Alkylsulfonyl oder ($C_1$-$C_2$)-Alkylcarbonyl,
$R^{14}$ und $R^{15}$ unabhängig voneinander Wasserstoff, ($C_1$-$C_6$)-Alkyl, Aryl oder Benzyl bedeuten.

**10.** Verwendung von Verbindungen der Formel (I) nach Anspruch 8, dadurch gekennzeichnet, daß
$R^1$ Wasserstoff, Hydroxy, Methoxy, Fluor, Chlor, Chlor, Brom, Cyano, Nitro, Difluormethyl, Trifluormethyl, Chlormethyl, Trichlormethyl, 2,2,2-Trifluorethyl oder Pentafluorethyl,
$R^2$ Wasserstoff, Fluor, Chlor, Hydroxy, Propyl, Ethyl, Methyl, Methoxy, Ethoxy, Difluormethyl, Trifluormethyl, Difluormethoxy, Trifluormethoxy, Methoxycarbonyl oder Ethoxycarbonyl,
$R^3$ Wasserstoff, hydroxy, Fluor, Chlor, Brom, Nitro, Difluormethyl, Trifluormethyl, Chlormethyl, Trichlormethyl, 2,2,2-Trifluorethyl, Pentafluorethyl, Difluormethoxy, Trifluormethoxy, Methylsulfonyl oder Ethylsulfonyl,
Q einen Rest aus der Gruppe mit der Formel

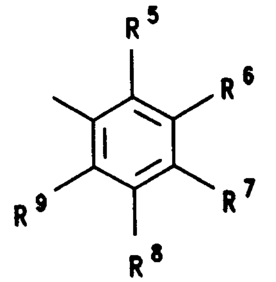

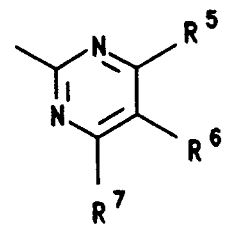

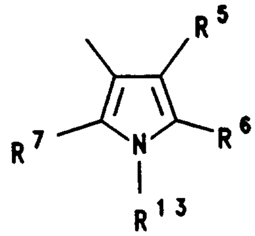

oder

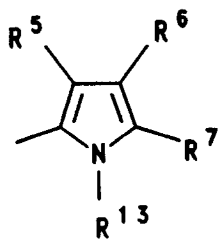

wobei

$R^5$, $R^6$, $R^7$, $R^8$ und $R^9$ unabhängig voneinander Wasserstoff, Fluor, Chlor, Brom, Cyano, Nitro, Hydroxy, Hydroxysulfonyl, Ethoxysulfonyl, Ethylaminosulfonyl, Methylaminosulfonyl, Carboxyl, Ethoxycarbonyl, Methoxycarbonyl, Ethylaminocarbonyl, Methylaminocarbonyl, Ethoxycarbonyloxy, Methoxycarbonyloxy, methylcarbonyloxy, t-Butyl, i-Propyl, Propyl, ethyl, Methyl, Difluormethyl, Trifluormethyl, Chlormethyl, Trichlormethyl, Methoxy, Ethoxy, Difluormethoxy oder Trifluormethoxy,

$R^{10}$, $R^{11}$ und $R^{12}$ unabhängig voneinander Wasserstoff, Fluor, Chlor, Brom Ethyl, Methyl, Difluormethyl, Trifluormethyl, Chlormethyl, Trichlormethyl, Methoxy, Ethoxy, Difluormethoxy oder Trifluormethoxy,

$R^{13}$ Wasserstoff, Phenylsulfonyl, Methylsulfonyl, Ethylsulfonyl, Methylcarbonyl oder Ethylcarbonyl,

$R^{14}$ und $R^{15}$ unabhängig voneinander Wasserstoff, Butyl, Propyl, Ethyl, Methyl oder Benzyl bedeuten.

**11.** Herbizide Mittel, dadurch gekennzeichnet, daß sie Verbindungen der Formel (I), definiert in einem der Ansprüche 8 bis 10, sowie übliche Formulierungshilfsmittel enthalten.

**12.** Verfahren zur Herstellung von Verbindungen der Formel (I), definiert in Anspruch 8, dadurch gekennzeichnet, daß man Säurechloride der Formel (II)

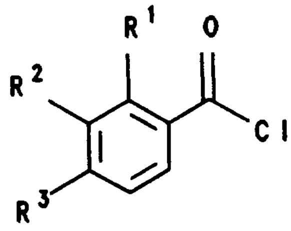

in denen $R^1$, $R^2$ und $R^3$ wie in Formel (I) definiert sind, mit aromatischen und heteroaromatischen Verbindungen der Formel (III)

Q - H (III)

in denen Q wie in Formel (I) definiert ist, in Gegenwart einer Lewissäure wie Aluminiumchlorid, Titantetrachlorid oder Bortrifluorid in einem chlorierten Kohlenwasserstoff wie Dichlormethan, Trichlormethan oder 1,1,2,2-Tetrachlorethan umsetzt.

**13.** Verfahren zur Herstellung eines Mittels gemäß Anspruch 12, dadurch gekennzeichnet, daß man ein oder mehrere Verbindungen der Formel (I) analog einer üblichen Pflanzenschutzformulierung aus der Gruppe, enthaltend Spritzpulver, wasserlösliche Pulver, emulgierbare Konzentrate, wäßrige Lösungen oder Konzentrate, Emulsionen, versprühbare Lösungen (Tank-mix), Kapselsuspensionen, Dispersionen auf Öl- oder Wasserbasis, Suspoemulsionen, Suspensionskonzentrate, Stäubemittel, ölmischbare Lösungen, Beizmittel, Mikro-, Sprüh-, Aufzugs- und Adsorptionsgranulate, Boden- und Streugranulate, wasserdispergierebare Granulate, ULV-Formulierungen, Mikrokapseln und Wachse formuliert.

**14.** Verfahren zur Bekämpfung von unerwünschten Pflanzen, dadurch gekennzeichnet, daß man auf diese oder auf die Flächen, auf denen die Pflanzen wachsen eine wirksame Menge einer Verbindung der Formel (I), definiert nach einem der Ansprüche 8 bis 10 appliziert.

**15.** Verfahren gemäß Anspruch 14, dadurch gekennzeichnet, daß unerwünschte Pflanzen in Nutzpflanzenkulturen bekämpft werden.

**16.** Verfahren gemäß Anspruch 15, dadurch gekennzeichnet, daß die Nutzpflanzenkultur eine aus der Gruppe Weizen, Gerste, Roggen , Reis und Mais ist.

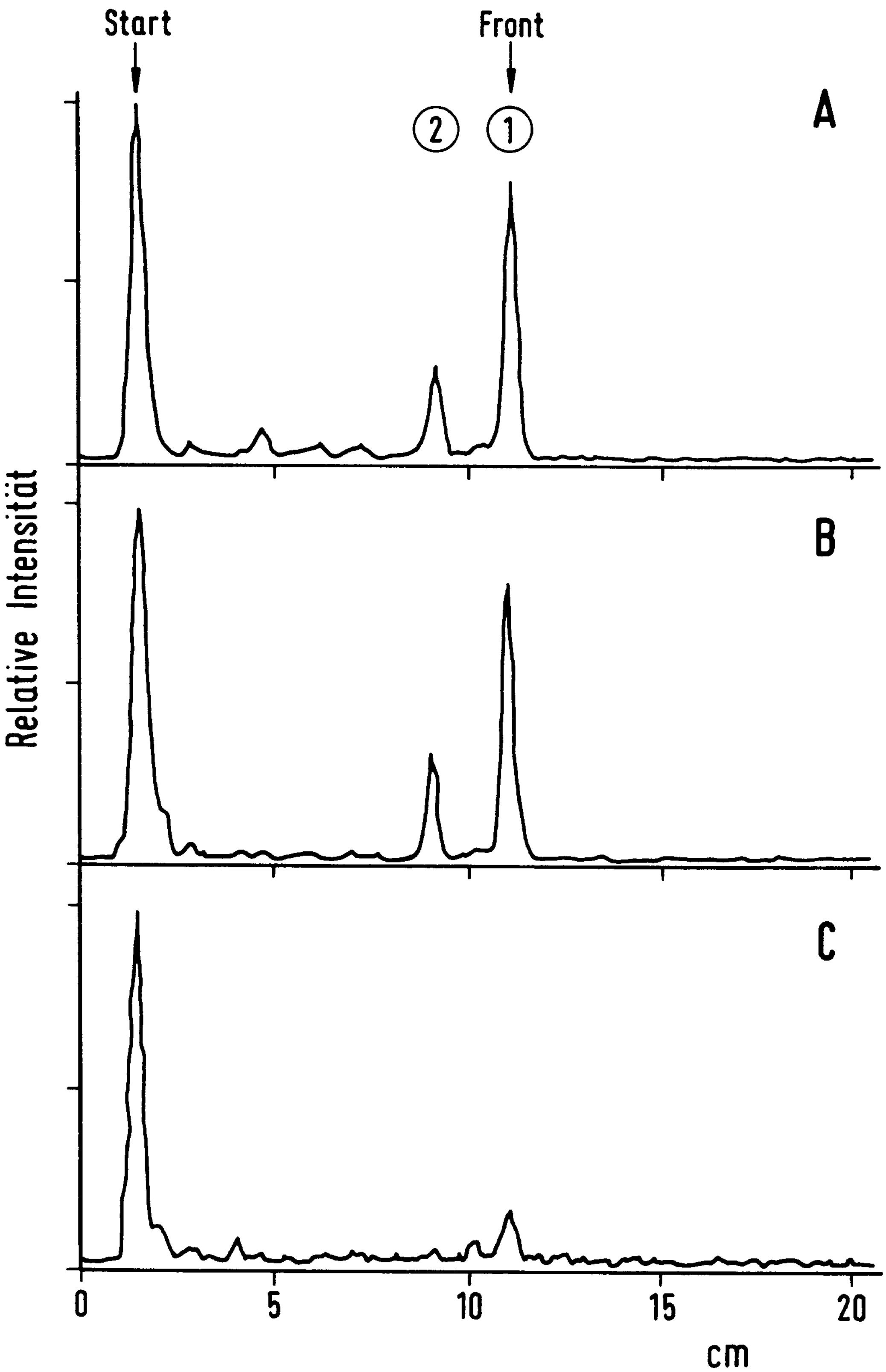


Fig. 1

Fig. 2

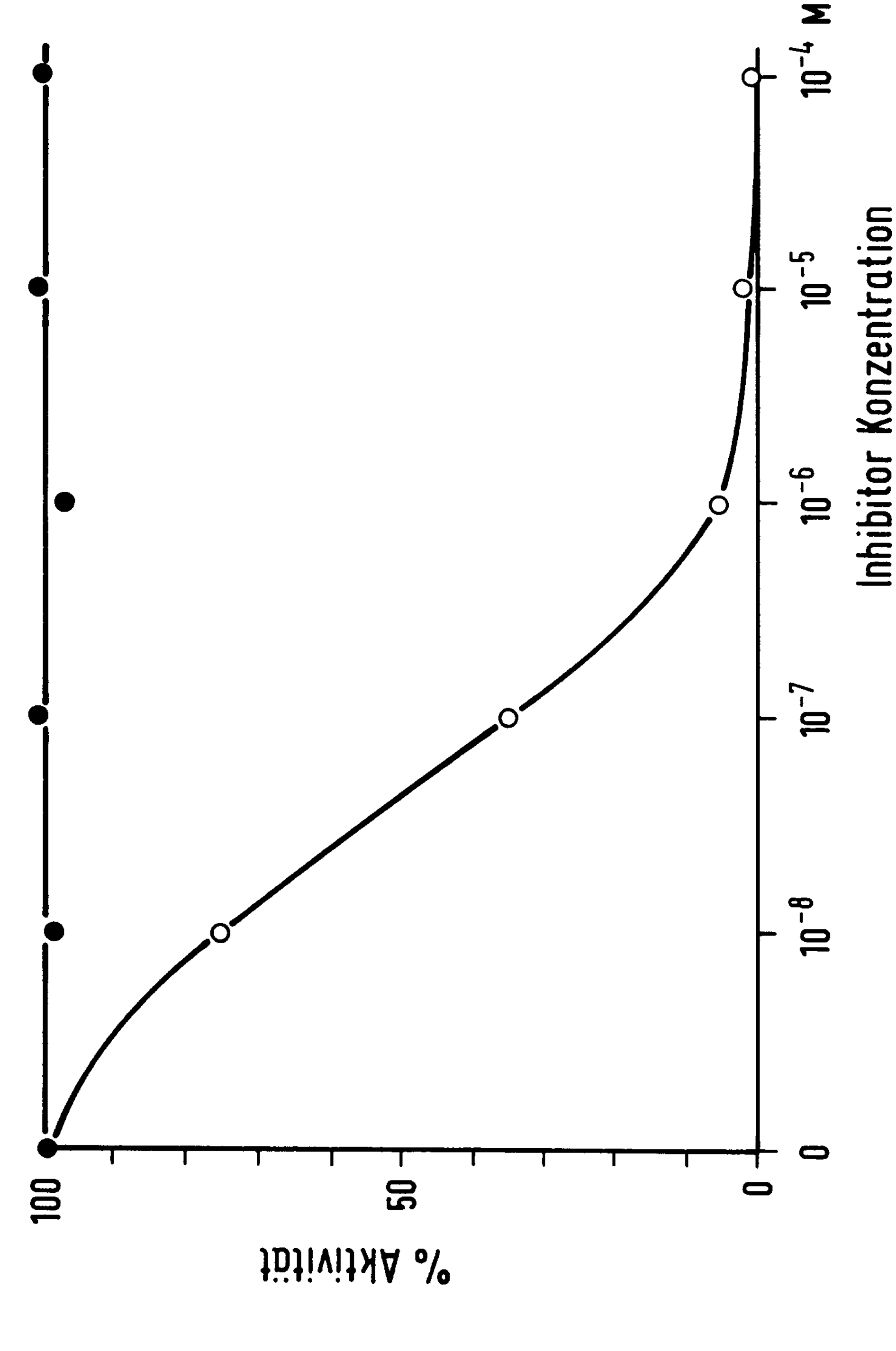
% Aktivität
100
50
0
0
$10^{-8}$
$10^{-7}$
$10^{-6}$
$10^{-5}$
$10^{-4}$ M
Inhibitor Konzentration